# EUROPEAN PATENT SPECIFICATION

(11) **EP 0 820 468 B1**
(45) Date of publication and mention of the grant of the patent: **28.06.2000**
(21) Application number: 96913491.5
(22) Date of filing: 09.04.1996
(51) Int. Cl.: C07K 14/505

(54) **PROCESS FOR THE PURIFICATION OF GLYCOPROTEINS LIKE ERYTHROPOIETIN**
VERFAHREN ZUR REINIGUNG VON GLYKOPROTEINEN WIE ERYTHROPOIETIN
PROCEDE DE PURIFICATION DES GLYCOPROTEINES TELLES QUE L'ERYTHROPOIETINE

(30) Priority: 14.04.1995 EP 95200945; 07.06.1995 US 475260
(43) Date of publication of application: 28.01.1998
(73) Proprietor: GRUPPO LEPETIT S.P.A., 20020 Lainate (MI) (IT)
(72) Inventor: ZANETTE, Dino, I-31010 Pianzano (IT); SARUBBI, Edoardo, Giacomo, F-94120 Fontenay-sous-Bois (FR); SOFFIENTINI, Adolfo, I-20099 Sesto San Giovanni (IT); RESTELLI, Ermenegildo, I-21040 Gerenzano (IT); GRIGOLETTO, Armando, I-27036 Mortara (IT)
(74) Representative: Macchetta, Francesco
(86) International application number: EP9601509
(87) International publication number: WO9632413

(56) References cited:
- EP-A- 0 358 463
- WO-A-86/04068
- GB-A- 2 024 829
- US-A- 4 568 488
- J. CHROMATOGR. (1988), 426(2), 376-80 CODEN: JOCRAM;ISSN: 0021-9673, XP000579995 DE CRISTOFARO, RAIMONDO ET AL: "Human platelet glycocalicin purification by phenyl boronate affinity chromatography coupled to anion-exchange high-performance liquid chromatography"
- PROTIDES BIOL. FLUIDS (1985), 33, 651-4 CODEN: PBFPA6;ISSN: 0079-7065, XP000600817 DUCROCQ, R. ET AL: "Separation of nonenzymically glycosylated proteins by phenylboronate affinity chromatography"

## Description

The present invention is directed to a simple and efficient process for the recovery of a biologically active glycoprotein from a biological fluid which contains it.

More particularly, the process of the invention is suitable for recovering highly glycosylated proteins, i.e. glycoproteins and glycopeptides having a sugar content higher than about 30% of their molecular weight.

A specific and preferred example of glycoprotein that can be conveniently purified according to the process of the invention is erythropoietin.

The preparation of erythropoietin from various sources, including genetically engineered cells, is reported in several European patent applications or patents, such as EP-148605, EP-205564, EP-209539, EP-267678, EP-649464 and EP-672160.

As used herein, the term "erythropoietin" or "erythropoietin product" is intended to include naturally occurring erythropoietin, urinary derived human erythropoietin as well as non-naturally occurring polypeptides having an amino acid sequence and glycosylation sufficiently duplicative of that of naturally occurring erythropoietin to allow possession of in vivo biological properties causing bone marrow cells to increase production of reticulocytes and red blood cells and encompasses also erythropoietin obtained from mammalian cell culture fluids upon introduction of an intact erythropoietin coding gene into the mammalian cells or erythropoietin obtained from mammalian cell culture fluids upon activation of the endogenous erythropoietin gene(s), including "gene activated" erythropoietin such as that described in international patent application publication no.WO 93/09222, which was filed as PCT/US 92/09627, designating also the US, or WO 94/12650, claiming the priority of US s.n. 07/985,586, that are herewith incorporated by reference.

The process of the invention is particularly suitable for the recovery of erythropoietin contained in a culture fluid of an erythropoietin producing cell culture.

As used herein, the term "culture fluid" is preferably intended to refer to any fluid of artificial origin, such as the cell culture fluid of mammalian cells and in particular of genetically transformed mammalian cells.

Preferably, the "culture fluid" referred to in this application is a culture fluid that has been separated from cells and cell debris by filtration or ultrafiltration, as conventional in this art. Moreover, in the present disclosure, the term "filtered culture fluid" is used to indicate a culture fluid that has been separated from cells and cells debris by filtration or ultrafiltration, as conventional in this art.

"Producing cells" are preferably stabilized or non-stabilized cell lines of eukaryotic or preferably of mammalian origin that are capable, upon cultivation in a suitable medium, of producing the desired glycoprotein in a recoverable amount. Representative examples of these cells include fibroblasts, keratinocytes, epithelial cells (e.g., mammary epithelial cells, intestinal epithelial cells), endothelial cells, glial cells, neural cells, formed elements of the blood (e.g. lymphocytes, bone marrow cells), muscle cells and precursors of these somatic cell types. As mentioned, these cells are preferably of mammalian origin (e.g., mouse, rat, rabbit, cat, dog, pig, cow, bird, sheep, goat, horse, monkey, human) and most preferably they are from primates and humans. In particular, the term "producing cells" encompasses also transfected primary, secondary, and immortalized cells of vertebrate origin, particularly of mammalian origin, and most preferably of primate or human origin transfected with exogenous genetic material that directly or indirectly causes the cells to produce recoverable amounts of erythropoietin such as those described in the above mentioned international patent application publication no.WO 93/09222, or WO 94/12650.

Examples of immortalized human cell lines useful for producing erythropoietin are widely known and available and include, but are not limited to, HeLa cells and derivatives of HeLa cells (such as ATCC CCL 2, 2.1 and 2.2), MCF-7 breast cancer cells (such as ATCC HTB 22), K-562 leukemia cells (such as ATCC CCL 234), KB carcinoma cells (such as ATCC CCL 17), 2780AD ovarian carcinoma cells [Van der Blick, A.M. et al., Cancer Res; 48:5927-5932 (1988)], Raji cells (ATCC CCL 86), Jurkat cells (ATCC TIB 152), Namalwa cells (ATCC CRL 1432), HL-60 cells (ATCC CCL 240), WiDr cells (ATCC CCL218), HT1080 cells (ATCC CCL 1219, Daudi cells (ATCC CCL213), RPMI 8226 cells (ATCC CCL 155), U-937 cells (ATCC CRL 1593), Bowes Melanoma cells (ATCC CRL 9607), WI-38VA13 subline 2R4 cells (ATCC CCL 75.1) and MOLT-4 cells (ATCC CRL 1582). Secondary human fibroblast strains, such as WI-38 (ATCC CCL 75) and MRC-5 (ATCC CCL 171) may also be used.

As mentioned, the process of the invention is suitable for recovering the desired glycoprotein from any such culture fluid and producing cells.

The extensive application of genetic engineering technologies to the large scale preparation of biologically active proteins has substantially enhanced the prospects of obtaining them in quantities that can satisfy their high demand. In several instances, however, their recovery from the culture fluid is troublesome, cumbersome and expensive. This is particularly the case when the protein is highly glycosylated, as mentioned above. Particularly in these instances, there continues to exist a need in the art for simple and efficient recovery procedures that are suitable for large scale production.

When the specific activity of erythropoietin is referred to in this specification, it is intended to be determined according to an ELISA method (e.g. the one reported in this application: Human Erythropoietin ELISA method, R & D systems, Inc.), while the protein content of the samples is measured by the BCA Protein Assay. These analysis methods are reported in more details hereinbelow under the heading: "Analysis methods".

One object of the present invention is therefore to provide a suitable chromatographic step, in an overall purification process, which employs dihydroxyboronyl bearing chromatographic matrices. This chromatographic step, because of its efficiency and versatility, can be the first one or a subsequent one in a multistep procedure. As a first chromatographic step, dihydroxyboronyl chromatography is preferably coupled with an ion exchange chromatographic step, as described below in more details, while as a subsequent chromatographic step it can be introduced at any stage of a multistep procedure.
This multistep procedure may suitably include additionally any of a number of chromatographic steps comprising ion exchange, size exclusion, hydrophobic interaction and affinity chromatography steps.

As a subsequent chromatography step in a multistep procedure, dihydroxyboronyl chromatography is preferably conducted by applying a material containing erytrhopoietin which is a semi-purified material to a pre-equilibrated dihydroxyboronyl bearing chromatographic matrix. The semi-purified material is also equilibrated in the matrix equilibrating buffer before applying it to the matrix. This equilibrating buffer is indicated heretofore as "first equilibrating buffer", and is defined below more in details. This process step is particularly suitable for the purification of erythropoietin.

One object of the present invention is a purification procedure which comprises:
a) contacting a filtered culture fluid, containing the glycoprotein to be isolated, with a pre-equilibrated dihydroxyboronyl bearing chromatographic matrix,
b) eluting with a first elution buffer and contacting this eluate directly with an anion exchange matrix bearing quaternary ammonium functional groups, and
c) eluting the glycoprotein with a second elution buffer.

As will be apparent from the complete reading of the present disclosure, these process steps allow a several-fold purification of a glycoprotein contained in a culture medium, therefore they are effectively used as a first purification step in a multistep procedure.

According to a preferred embodiment of the process of the invention, a culture fluid containing the glycoprotein to be isolated is submitted to a procedure which comprises:
a) contacting a filtered culture fluid with a dihydroxyboronyl bearing chromatographic matrix,
b) eluting with a first elution buffer and contacting this eluate directly with an anion exchange matrix bearing quaternary ammonium functional groups,
c) eluting with a second elution buffer,
d) ultrafiltering on a 5,000-30,000 D cutoff membrane,
e) optionally diafiltering to exchange the buffer to the one suitable for the next step, and
f) gel filtering on a separation resin having a separation range between 5,000 and 250,000 D.

According to another preferred embodiment, the process of the invention comprises:
a) contacting a filtered culture fluid adjusted to pH 7.5-9.0 with a dihydroxyboronyl bearing chromatographic matrix equilibrated with a first equilibrating buffer which is an aqueous buffer at a concentration of 25-100 mM, with an ionic strength between 2 and 20 mS/cm² and a pH between 7.5 and 9.0,
b) washing subsequently with the first equilibrating buffer and then with the first equilibrating buffer containing from 10 to 100 mM of a 1,2-cis-diol containing low molecular weight substance,
c) eluting with a first elution buffer which is an aqueous buffer having a pH between 7.5 and 11.0 containing a compound having 1-hydroxy-2-amino groups at a concentration of 20-200 mM, possibly in the presence of a chaotropic agent at 2 to 8M, a cyanate acceptor at 2-40 mM, and a surfactant from 0.01% to 0.1% (w/w),
d) contacting this eluate with an anion exchange matrix bearing quaternary ammonium functional groups equilibrated in a second equilibrating buffer which is an aqueous buffer having a pH between 7.5 and 11.0 and containing from 0.01% to 0.1% (w/w) of a surfactant,
e) washing subsequently with the second equilibrating buffer and the same buffer containing additionally up to 50 mM salt,
f) eluting with a second elution buffer having a pH between 7.5 and 11.0 in the presence of from 0.01% to 0.1% (w/w) of a surfactant and from 150 to 350 mM salt,
g) ultrafiltering on a 5,000-30,000 D cutoff membrane,
h) optionally diafiltering to exchange the buffer to one that is suitable for the next gel filtration step, and
i) gel filtering on a separation resin having a separation range between 5,000 and 250,000 D.

"Dihydroxyboronyl bearing chromatographic matrices" refer to any known chromatographic matrix bearing a boronate function wherein the boron atom is stably bound to a carbon atom chain wherein the proximal carbon atom provides enough free electron density to remain bound to boron under the various conditions of use. Most preferably, this proximal carbon atom is an aromatic carbon atom, for example a carbon atom belonging to a possibly substituted benzene ring. Particularly preferred for use in the process of the invention are the so-called phenylboronate resins. Examples of these resins are reported in US patents 4,562,251, 4,778,888 and 4,269,605. Among them, phenyl boronate agarose matrices are currently the most preferred. These matrices are at present commercially available, e.g. from Amicon Inc.or Grace Inc., under the trade name MATREX GEL, including MATREX GEL PBA-10, PBA-30 and PBA-60. Currently, the most preferred phenyl boronate agarose for the application as a first purification step is the one sold as MATREX GEL PBA-60, which is the one wherein m-aminophenyl boronic acid is covalently coupled with agarose having a size range of 50-150 µm diameter spherical beads with a ratio of 60-100 µM boronic acid/ml gel. As a subsequent purification step, however, a phenyl boronate agarose matrice such as MATREX GEL PBA-30 is preferred, which is the one wherein m-aminophenyl boronic acid is covalently coupled with agarose having a size range of 50-150 µm diameter spherical beads with a ratio of 30-50 µM boronic acid/ml gel.

Currently, the most preferred use of these chromatographic matrices is in column chromatography systems, even if batchwise or other systems are not entirely ruled out.

When used in column chromatography systems, the dihydroxyboronyl bearing chromatographic matrix is preferably used at room temperature, preferably between 5 and 25°C, and most preferably between 10 and 20°C, with about 15°C being the most preferred temperature.

The above mentioned "first equilibrating buffer" is preferably selected from glycine, phosphate, trialkylammonium bicarbonate and 4-(2-hydroxyethyl)-1-piperazinoethane sulfonic acid (HEPES), at the indicated concentration range, ionic strength and pH. A particularly preferred concentration for this first equilibrating buffer is a concentration of about 50mM, while a particularly preferred pH value is about 8.5, and a preferred conductivity is of about 2.5 mS/cm². These are preferred conditions both in case of first step and subsequent step purification.

The above mentioned "1,2-cis-diol containing substance" is any of the known low molecular weight compounds having 1,2-cis-diol functional groups, i.e. having at least two hydroxy groups on adjacent carbon atoms that are held in or can assume a coplanar or quasi-coplanar configuration. Representative examples of these compounds, that are in any case commonly known in the art, are small open-chain polyols such as sorbitol, mannitol, adonitol, arabitol, glycerol, erythritol, and cis-inositol, and closed-chain monosaccharides such as ribose and mannose, with sorbitol being currently the most preferred. Particularly preferred is the use of a 1,2-cis-diol containing low molecular weight substance at a concentration of about 50 mM in the case of a first purification step, while in a subsequent purification step a concentration of 20-100 mM is currently preferred, with 100 mM being the most preferred one.

The above mentioned "compound having 1-hydroxy-2-amino groups" which is the main constituent of said "first elution buffer" is any compound having such functional groups and capable of forming an aqueous buffer in the pH range indicated above. Representative examples of any such compounds are: 2-amino-2-hydroxymethyl-1,3-propanediol, which is known also as TRIS or tris(hydroxymethyl); aminomethane; bis(hydroxymethyl)aminoethane; N-[2-hydroxy-1,1-bis(hydroxymethyl)ethyl]glycine, which is known also as tricine; and N,N-bis(2-hydroxyethyl)glycine, which is known also as bicine. They are preferably employed at a concentration of about 20-100 mM, with about 50mM being currently most preferred, in the case of a first purification step. Preferably, the first elution buffer is adjusted to a pH of about 8.5.

For the application as a subsequent purification step, the preferred concentration range is 20-150 mM, with 100 mM being most preferred. Also for this application, TRIS is the currently preferred one.

Preferred elution conditions where phenylboronate chromatography is used as a second chromatographic step involve an elution buffer which includes TRIS 100 mM and sorbitol 100 mM.

Preferably, the "second equilibrating buffer" and the "second elution buffer" have the above defined compound having 1-hydroxy-2-amino groups as their main component. In this case, its preferred concentration is between 20 and 200 mM.

A "chaotropic agent" is an agent favoring the salting-in of a proteinaceus material, and thus its solubilization in an aqueous medium, generally because of its dissociating properties.

Representative examples of chaotropic agents are urea and its derivatives and guanidine.
The chaotropic agent, if present, is employed in a concentration between 2 and 8M, with about 4-6M being preferred.

A cyanate acceptor is any of the known substances capable of readily binding CNO⁻ ions that may form as a result of the hydrolysis of urea.
Glycine is preferred as a cyanate acceptor. For its use in the process of the invention, glycine is preferably employed at a concentration from 2 to 40 mM and most preferably at about 20 mM.

The term "surfactant", as used herein refers to any of the known substances that reduce the surface tension, i.e. the force that acts at the surface of liquids to reduce their surface area. Preferred examples of surfactants are the polyoxyethylene sorbitan derivatives of fatty acids known as "Tween®" with "Tween 20" (i.e. polyoxyethylene sorbitan monolaurate) being currently preferred. Currently, the surfactant, if present, is preferably employed at a concentration of about 0.01-0.1% (w/w), with about 0.01% being currently most preferred.

The above mentioned anion exchange matrix bearing quaternary ammonium functional groups is any of the known and commercially available anion exchange matrices having said functional groups. Preferred for use in the process of the invention are agarose or cellulose based matrices, such as microcrystalline cellulose or crosslinked agarose. Also particularly preferred are those matrices bearing diethyl aminoethyl, triethyl aminomethyl or trimethyl aminomethyl functional groups.
A particularly preferred anion exchange matrix is trimethyl aminomethyl crosslinked agarose, which is commercially available e.g. as Q-Sepharose® (Pharmacia AB). The chromatographic step involving these matrices is most preferably conducted as a column chromatography which is conducted at room temperature.

When a salt is added to a washing or elution buffer as reported above, it is added to increase the ionic strength of the buffer, as conventional in the art. Any of the salts conventionally used in the art can be employed in the process of the invention for this purpose, with NaCl being one of those most frequently and conveniently used.

The ultrafiltration step is conducted as conventional in the art, using a tangential flow system or a stirred cell system. Preferably, the membrane is a polysulfonic membrane or regenerated cellulose membrane of the types commercially available e.g. from Millipore Inc. or Amicon Inc. Currently preferred for use in the process of the invention are those membranes having a cutoff of 10,000.
The ultrafiltrate is then optionally submitted to diafiltration according to procedures known per se in the art, preferably in the same buffer that is being used for the next gel filtration step. This gel filtration buffer is an aqueous buffer having a pH between 6 and 8. The salt is present preferably from 100 to 200 mM in this buffer and still more preferably, the salt is 100mM and the pH is about 7.4-7.5.

The conventional gel filtration matrices can be conveniently used in this process step. Representative examples of these matrices are polydextranes crosslinked with acrylamides, such as high mechanical strength composite hydrophilic gels prepared by covalently crosslinking allyl dextran with N,N'-methylene bisacrylamide and crosslinked cellulose gels.
Commercially available crosslinked dextrane-acrylamides are known under the trade name SEPHACRYL and are available from Pharmacia AB. A preferred SEPHACRYL gel is SEPHACRYL S-200 HR, which has a bead size of 25-75 µm and its crosslinking is controlled so that it has a fractionation range for globular protein of 5,000-250,000 D.
Examples of crosslinked cellulose gels are those commercially available crosslinked porous cellulose gels, e.g. GLC 300 (average particle size 44-125 µm) or GLC 1,000 (average particle size 53-125 µm) that are available from Amicon Inc.

The process of the invention is particularly apt to large scale production, since it consists of a sequence of steps that are easily scalable without the major drawbacks of the known ones.
Moreover, it employs only aqueous solvents, and this represents a further advantage, as it is appreciated by those skilled in the art.
Additionally, it allows passing from its first chromatographic step (i.e. the one with the dihydroxyboronyl matrix) to its second chromatographic step (i.e. the one with the anion exchange matrix) by directly transferring the eluate from the first step to the second one, without requiring any solvent exchange or adjustment.
In addition to making the overall operations easier, this feature of the process of the invention contributes to improving the recovery yields.

The process of the invention in fact allows the recovery of an erythropoietin product having an average specific activity of 145,000-175,000 ELISA units/mg, starting from a filtered cell fluid with an average specific activity of 500-2,000 ELISA units/mg.

Moreover, the combined first and second chromatographic step allow a 60-100 fold purification of the initial culture fluid, with an overall purification factor of 110-150 fold for the complete process.

As in the case of erythropoietin, the glycoprotein product recovered at the end of the process of the invention may be isolated as a solid after salt removal and lyophilization as conventional in the art, or the obtained solution can be solvent exchanged, e.g. by diafiltration, to have a solution suitable for pharmaceutical formulation, such as one of those described in EP 430200.

The following examples are illustrative only and are not to be construed to limit the scope of the claimed invention.

### EXAMPLE 1

### Phenyl boronate chromatography as a first purification step in erythropoietin purification.

The supernatant of a culture of a human erythropoietin producing cell obtained as described in example 21 of WO 93/09222 or in WO 94/12650 containing approximately 20 mg of erythropoietin is filtered on a mixed membrane cartridge (1.2 and 0.5 µm; Opticap, Millipore Inc) and then ultrafiltered on a 30,000 D regenerated cellulose spiral cartridge S1Y30 (Amicon Inc) and diafiltered with water and 0.05M HEPES pH 8.5, conductivity 2.5 mS/cm². The ultrafiltrate is loaded on a phenylboronate agarose chromatographic column (120 ml swollen resin; PBA 60, Amicon Inc) equilibrated with 0.05M HEPES pH 8.5 containing 0.01% Tween 20 (Buffer A) and kept at about 10-15 °C by water refrigeration. The flow rate is set at about 2-4 column volumes (CV) / h. After having washed with Buffer A until the baseline stabilizes [optical density (OD) at 280 nm], washing is continued with 0.05M HEPES pH 8.5 containing 0.01% Tween 20 and 0.05M sorbitol (Buffer B). Elution is carried out with 0.02M TRIS pH 8.5 containing 6M urea, 0.02M glycine and 0.01% Tween 20 (Buffer C) and the peak (OD at 280 nm) that elutes is collected (about 4-5 CV; yield 70-80%, purification factor about 20-fold) and directly loaded onto a trimethyl aminomethyl crosslinked agarose anion exchange resin (25 ml swollen resin; Q-Sepharose FF, Pharmacia AB) equilibrated with 0.02M TRIS pH 8.5 containing 0.01% Tween 20 (Buffer D). After washing, at a constant flow rate, with Buffer D until baseline stabilizes, and then with Buffer D containing also 0.05 M NaCl (Buffer E), elution is done with 0.02M TRIS pH 8.5 containing 0.01% Tween 20 and 0.15 M NaCl (Buffer F). The peak (OD 280 nm) is collected (recovery about 80%) and concentrated by ultrafiltration with a stirred cell having a membrane of 10,000 D cutoff (YM10, Amicon).

The concentrated solution is loaded on a crosslinked dextrane-acrylamide gel filtration column (Sephacryl S 200; Pharmacia AB) equilibrated with 0.02M TRIS pH 7.4 containing 0.15 M NaCl (Buffer G) and eluted with the same buffer. Fractions are collected and those containing the erythropoietin product (ELISA assay) are pooled and concentrated by ultrafiltration as described above.

### EXAMPLE 2

### Phenyl boronate chromatography as a subsequent purification step in erythropoietin purification

A solution of semi-purified human erythropoietin (from producing cell obtained as described in example 21 of WO 93/09222 or in WO 94/12650) containing approximately 20 mg of erythropoietin is diafiltered or dialyzed with water and 0.05M HEPES pH 8.5, and 0.15M NaCl, conductivity about 18 mS/cm², and loaded on a phenylboronate agarose chromatographic column (120 ml swollen resin; PBA 30, Amicon Inc) equilibrated with 0.05M HEPES pH 8.5 containing 0.15M NaCl (Buffer A') and kept at about 10-15 °C by water refrigeration. The flow rate is set at about 2-4 column volumes (CV) / h. After having washed with Buffer A' until the baseline stabilizes [optical density (OD) at 280 nm], elution is carried out with 0.10M TRIS pH 8.5 containing 0.1M sorbitol (Buffer B') and the peak (OD at 280 nm) that elutes is collected (about 4-5 CV; yield about 90%).

### Analysis methods:

### ELISA:

The specific activity of the erythropoietin product obtained according to the process described above is measured by the Human EPO ELISA method that is known and commercially available as QUANTIKINE IVD (R&D SYSTEMS Inc).

### Protein assay:

Protein assay is conducted by the BCA Protein Assay Reagent method (Pierce Chemical Co) that is a method that combines the biuret reaction (protein reduction of Cu²⁺ to Cu¹⁺ in an alkaline medium) with the highly specific reaction of bicinchoninic acid (BCA) for Cu¹⁺.

### Specific activity:

Specific activity for samples is calculated by the following formula: Specific activity=Total Units of erythropoietin by ELISA/Total mg protein by BCA assay. A purified preparation of erythropoietin has a specific activity of about 200,000 ELISA units/mg protein by BCA assay. Specific activity using the ELISA to quantify units of erythropoietin is only indirectly linked to the actual biological activity of erythropoietin. A test generally performed to determine in vivo specific activity is the exhypoxic polycythemic mouse bioassay as is known by those skilled in the art (such an assay can determine the specific activity as IU/mg, as known by one skilled in the art).

### HPLC analysis:

Instrument: Waters 616 LC System equipped with a PDA detector model 996
Column: Vydac protein C₄, 3 µm, 4.6 x 250 mm
Mobile phase: A) 0.1% aqueous trifluoroacetic acid : acetonitrile, 95:5, B) 0.1% aqueous trifluoroacetic
acid : acetonitrile, 5:95
Gradient: min (%B): 5 (5), 50 (95)
Flow rate: 1 ml/min

### SDS-PAGE analysis:

SDS-PAGE analysis is done on a Phast System, Pharmacia AB and the densitometry measurements are done with a Flying Spot scanning densitometer CS-9301 PC, Shimadzu Co, according to the manufacturer's instructions and common knowledge in this field.

### Analytical data and results:

Having collected data from several runs, the specific activity of the erythropoietin product of the cell supernatant resulted in the range 500-2,000 ELISA units/mg; the specific activity of the erythropoietin product recovered after the first chromatographic step is about 35,000-95,000 ELISA units/mg; the specific activity of the erythropoietin product recovered at the end of the procedure described in Example 1 is, in the average, about 145,000 - 175,000 ELISA units/mg.

The HPLC analysis as well as the SDS-PAGE analysis conducted according to the methods reported above showed that the the erythropoietin product recovered at the end of the procedure had a principal peak which represented more than 92% of the area under the curve (AUC).

## Claims

1. A process for the purification of erythropoietin which comprises the following steps:
a) applying a material containing erythropoietin to a dihydroxyboronyl chromatographic matrix pre-equilibrated with a first equilibrating buffer,
b) washing with the equilibrating buffer and
c) eluting with an aqueous buffer having a pH between 7.5 and 11 containing a compound having 1-hydroxy-2-amino groups and a 1,2-cis-diol containing substance.

2. A process for the purification of erythropoietin which comprises:
a) contacting a filtered culture fluid, containing the biologically active glycoprotein to be isolated, with a dihydroxyboronyl bearing chromatographic matrix, pre-equilibrated with a first equilibrating buffer,
b) eluting with a first elution buffer and contacting this eluate directly with an anion exchange matrix bearing quaternary ammonium functional groups
c) eluting the glycoprotein with a second elution buffer and collecting it.

3. A process for the purification of erythropoietin which comprises:
a) contacting a filtered culture fluid, containing the glycoprotein to be isolated, with a dihydroxyboronyl bearing chromatographic support, pre-equilibrated with a first equilibrating buffer,
b) eluting with a first eluting buffer,
c) contacting this eluate directly with an anion exchange matrix bearing quaternary ammonium functional groups,
d) eluting with a second eluting buffer ,
e) ultrafiltering on a 5,000-25,000 D cutoff membrane,
f) optionally diafiltering to exchange the buffer to the one suitable for the next step, and
g) gel filtering on a separation resin having a separation range between 5,000 and 250,000 D.

4. A process for the purification of erythropoietin which comprises:
a) contacting a filtered culture fluid adjusted to pH 7.5-9.0 with a dihydroxyboronyl bearing chromatographic support equilibrated with a first equilibrating buffer which is an aqueous buffer at a concentration of 25-100 mM, with a ionic strength between 2 and 20 mS/cm² and a pH between 7.5 and 9.0,
b) washing subsequently with the first equilibrating buffer and then with the first equilibrating buffer containing from 10 to 100 mM of a 1,2-cis-diol containing low molecular weight substance,
c) eluting with a first eluting buffer which is an aqueous buffer having a pH between 7.5 and 11.0 containing a compound having 1-hydroxy-2-amino groups at a concentration of 20-200 mM, possibly in the presence of a chaotropic agent at 2 to 8M, a cyanate acceptor at 2-40 mM, and a surfactant from 0.02% to 0.1% (w/w),
d) contacting this eluate with an anion exchange matrix bearing quaternary ammonium functional groups equilibrated in a second equilibrating buffer which is an aqueous buffer having a pH between 7.5 and 11.0 and from 0.01t to 0.1% (w/w) of a surfactant,
e) washing subsequently with the second equilibrating buffer and the same buffer containing additionally up to 50 mM salt,
f) eluting with a second eluting buffer having a pH between 7.5 and 11.0 in the presence of from 0.01% to 0.1% (w/w) of a surfactant and from 150 to 350 mM salt,
g) ultrafiltering on a 5,000-30,000 D cutoff membrane,
h) optionally diafiltering to exchange the buffer to one that is suitable for the next gel filtration step, and
i) gel filtering on a separation resin having a separation range between 5,000 and 250,000 D.

5. A process according to any one of claims 1, 2, 3 or 4 wherein the dihydroxyboronyl bearing chromatographic support is a phenylboronate agarose.

6. A process according to any one of claims 1, 2, 3 or 4 wherein the first equilibrating buffer is selected from glycine, phosphate, trialkylammonium bicarbonate and 4-(2-hydroxyethyl)-1-piperazinoethane sulfonic acid.

7. A process according to claim 6 wherein the first equilibrating buffer is 4-(2-hydroxyethyl)-1-piperazinoethane sulfonic acid at a concentration of about 0.05 M.

8. A process according to any one of claims 1 to 7 wherein the pH of the first equilibrating buffer is about 8.5.

9. A process according to any one of claims 1 or 4 to 8 wherein the 1,2-cis-diol containing low molecular weight substance is selected from small open-chain polyols such as sorbitol, mannitol, adonitol, arabitol, glycerol, erythritol, and cis-inositol, and closed-chain monosaccharides such as ribose and mannose.

10. A process according to claim 9 wherein the 1,2-cis-diol containing low molecular weight substance is sorbitol.

11. A process according to claim 9 or 10 wherein the 1,2-cis-diol containing low molecular weight substance is present in a concentration of about 0.05 M.

12. A process according to any one of claims 1 or 4 to 11 wherein the second equilibrating buffer contains a compound having 1-hydroxy-2-amino groups at a concentration of 0.02 - 0.2 M.

13. A process according to any one of claims 1 or 4 to 11 wherein the second eluting buffer contains a compound having 1-hydroxy-2-amino groups at a concentration of 0.05 M.

14. A process according to any one of claims 4 to 13 wherein the compound having 1-hydroxy, 2-amino groups is selected from 2-amino-2-hydroxymethyl-1,3-propanediol, bis(hydroxymethyl)aminoethane, N-[2-hydroxy-1,1-bis(hydroxymethyl)ethyl]glycine, and N,N-bis(2-hydroxyethyl)glycine.

15. A process according to any one of claims 4 to 14 wherein the chaotropic agent is selected from urea and its derivatives, and guanidine.

16. A process according to claim 15 wherein the chaotropic agent is 6M urea.

17. A process according to any one of claims 4 to 16 wherein the cyanate acceptor is glycine.

18. A process according to any one of claims 3 to 17 wherein the anion exchange matrix bearing quaternary ammonium functional groups is selected from microcrystalline cellulose or crosslinked agarose matrices bearing diethyl aminoethyl, triethyl aminomethyl or trimethyl aminomethyl functional groups.

19. A process according to claim 18 wherein the anion exchange matrix is trimethyl aminomethyl agarose.

20. A process according to any one of claims 3 to 19 wherein the gel filtration is carried out with controlled pore crosslinked dextrane-acrylamide gel matrix.

## Patentansprüche

1. Verfahren zur Reinigung von Erythropoietin, umfassend die folgenden Schritte:
a) Aufbringen eines Erythropoietin enthaltenden Materials auf eine mit einem ersten äquilibrierenden Puffer preäquilibrierte chromatographische Dihydroxyboronyl-Matrix,
b) Waschen mit dem äquilibrierenden Puffer und
c) Eluieren mit einem wäßrigen Puffer mit einem pH-Wert zwischen 7,5 und 11, der eine Verbindung mit 1-Hydroxy-2-aminoresten und einen eine 1,2-cis-Diolgruppe enthaltenden Stoff enthält.

2. Verfahren zur Reinigung von Erythropoietin, umfassend:
a) Inkontaktbringen einer das zu isolierende biologisch aktive Glycoprotein enthaltenden filtrierten Kulturflüssigkeit mit einer mit einem ersten äquilibrierenden Puffer preäquilibrierten, Dihydroxyboronyl-Reste tragenden chromatographischen Matrix,
b) Eluieren mit einem ersten eluierenden Puffer und direktes Inkontaktbringen des Eluats mit einer quartäre Ammonium-funktionelle Reste tragenden Anionenaustauschermatrix,
c) Eluieren des Glycoproteins mit einem zweiten eluierenden Puffer und es Auffangen.

3. Verfahren zur Reinigung von Erythropoietin, umfassend:
a) Inkontaktbringen einer das zu isolierende Glycoprotein enthaltenden filtrierten Kulturflüssigkeit mit einem mit einem ersten äquilibrierenden Puffer preäquilibrierten, Dihydroxyboronyl-Reste tragenden chromatographischen Träger,
b) Eluieren mit einem ersten eluierenden Puffer,
c) direktes Inkontaktbringen dieses Eluats mit einer quartäre Ammonium-funktionelle Reste tragenden Anionenaustauschermatrix,
d) Eluieren mit einem zweiten eluierenden Puffer,
e) Ultrafiltrieren auf einer 5000-25000 D Cut-off-Membran,
f) gegebenenfalls Diafiltrieren zum Austausch des Puffers gegen einen für den nächsten Schritt geeigneten und
g) Gelfiltrieren auf einem Trennharz mit einem Trennbereich zwischen 5000 und 250000 D.

4. Verfahren zur Reinigung von Eythropoietin, umfassend:
a) Inkontaktbringen einer auf einen pH-Wert 7,5-9,0 eingestellten, filtrierten Kulturflüssigkeit mit einem mit einem ersten äquilibrierenden Puffer, der ein wäßriger Puffer mit einer Konzentration von 25-100 mM, einer Ionenstärke zwischen 2 und 20 mS/cm² und einem pH-Wert zwischen 7,5 und 9,0 ist, äquilibrierten, Dihydroxyboronyl-Reste tragenden chromatographischen Träger,
b) darauffolgendes Waschen mit dem ersten äquilibrierenden Puffer und anschließend mit dem ersten äquilibrierenden Puffer, welcher 10 bis 100 mM eines eine 1,2-cis-Diolgruppe enthaltenden niedermolekularen Stoffs enthält,
c) Eluieren mit einem ersten eluierenden Puffer, der ein wäßriger Puffer mit einem pH-Wert zwischen 7,5 und 11,0 ist, welcher eine Verbindung mit 1-Hydroxy-2-aminoresten in einer Konzentration von 20-200 mM enthält, möglicherweise in der Gegenwart eines chaotropen Agens mit 2 bis 8 M, eines Cyanat-Akzeptors mit 2-40 mM und eines grenzflächenaktiven Mittels von 0,02 % bis 0,1 % (w/w),
d) Inkontaktbringen dieses Eluats mit einer quartäre Ammonium-funktionelle Reste tragenden, in einem zweiten äquilibrierenden Puffer, der ein wäßriger Puffer mit einem pH-Wert zwischen 7,5 und 11,0 ist und von 0,01 % bis 0,1 % (w/w) eines grenzflächenaktiven Mittels enthält, äquilibrierten Anionenaustauschermatrix,
e) darauffolgendes Waschen mit dem zweiten äquilibrierenden Puffer und demselben Puffer, der zusätzlich bis zu 50 mM Salz enthält,
f) Eluieren mit einem zweiten eluierenden Puffer mit einem pH-Wert zwischen 7,5 und 11,0 in Gegenwart von 0,01 % bis 0,1 % (w/w) eines grenzflächenaktiven Mittels und von 150 bis 350 mM Salz,
g) Ultrafiltrieren auf einer 5000-30000 D Cut-off-Membran,
h) gegebenenfalls Diafiltrieren zum Austauschen des Puffers gegen einen, der für den nächsten Gelfiltrationsschritt geeignet ist und
i) Gelfiltrieren auf einem Trennharz mit einem Trennbereich zwischen 5000 und 250000 D.

5. Verfahren nach einem der Ansprüche 1, 2, 3 oder 4, in dem der Dihydoxyboronyl-Reste tragende chromatographische Träger eine Phenylboronat-Agarose ist.

6. Verfahren nach einem der Ansprüche 1, 2, 3 oder 4, in dem der erste äquilibrierende Puffer aus Glycin, Phosphat, Trialkylammoniumbicarbonat und 4-(2-Hydroxyethyl)-1-piperazinoethan-sulfonsäure ausgewählt wird.

7. Verfahren nach Anspruch 6, in dem der erste äquilibrierende Puffer 4-(2-Hydroxyethyl)-1-piperazinoethan-sulfonsäure mit einer Konzentration von etwa 0,05 M ist.

8. Verfahren nach einem der Ansprüche 1 bis 7, in dem der pH-Wert des ersten äquilibrierenden Puffers etwa 8,5 ist.

9. Verfahren nach einem der Ansprüche 1 oder 4 bis 8, in dem der eine 1,2-cis-Diolgruppe enthaltende niedermolekulare Stoff aus kleinen offenkettigen Polyolen wie Sorbit, Mannit, Adonit, Arabit, Glycerin, Erythrit und cis-Inosit, und geschlossenkettigen Monosacchariden, wie Ribose und Mannose ausgewählt wird.

10. Verfahren nach Anspruch 9, in dem der eine 1,2-cis-Diolgruppe enthaltende niedermolekulare Stoff Sorbit ist.

11. Verfahren nach Anspruch 9 oder 10, in dem der eine 1,2-cis-Diolgruppe enthaltende niedermolekulare Stoff in einer Konzentration von etwa 0,05 M anwesend ist.

12. Verfahren nach einem der Ansprüche 1 oder 4 bis 11, in dem der zweite äquilibrierende Puffer eine Verbindung mit 1-Hydroxy-2-aminoresten in einer Konzentration von 0,02-0,2 M enthält.

13. Verfahren nach einem der Ansprüche 1 oder 4 bis 11, in dem der zweite eluierende Puffer eine Verbindung mit 1-Hydroxy-2-aminoresten in einer Konzentration von 0,05 M enthält.

14. Verfahren nach einem der Ansprüche 4 bis 13, in dem die Verbindung mit 1-Hydroxy-2-aminoresten aus 2-Amino-2-hydroxymethyl-1,3-propandiol, Bis-(hydroxymethyl)aminoethan, N-[2-Hydroxy-1,1-bis(hydroxymethyl)ethyl]glycin und N,N-bis(2-hydroxyethyl)glycin ausgewählt wird.

15. Verfahren nach einem der Ansprüche 4 bis 14, in dem das chaotrope Agens aus Harnstoff und seinen Derivaten und Guanidin ausgewählt wird.

16. Verfahren nach Anspruch 15, in dem das chaotrope Agens 6 M Harnstoff ist.

17. Verfahren nach einem der Ansprüche 4 bis 16, in dem der Cyanat-Akzeptor Glycin ist.

18. Verfahren nach einem der Ansprüche 3 bis 17, in dem die quartäre Ammonium-funktionelle Reste tragende Anionenaustauschermatrix aus mikrokristalliner Cellulose oder vernetzten Agarosematrices, welche Diethylaminoethyl-, Triethylaminomethyl- oder Trimethylaminomethyl-funktionelle Reste tragen, ausgewählt wird.

19. Verfahren nach Anspruch 18, in dem die Anionenaustauschermatrix Trimethylaminomethyl-Agarose ist.

20. Verfahren nach einem der Ansprüche 3 bis 19, in dem die Gelfiltration mit einer vernetzten Dextran-Acrylamid-Gelmatrix mit festgelegter Porengröße durchgeführt wird.

## Revendications

1. Procédé pour la purification d'érythropoïétine, qui comprend les étapes suivantes :
a) application d'un matériau contenant de l'érythropoïétine à une matrice chromatographique au dihydroxyboronyle équilibrée au préalable avec un premier tampon d'équilibrage,
b) lavage avec le tampon d'équilibrage, et
c) l'élution avec un tampon aqueux ayant un pH entre 7,5 et 11 contenant un composé ayant des groupes 1-hydroxy-2-amino et une substance contenant un 1,2-cis-diol.

2. Procédé pour la purification d'érythropoïétine, qui comprend :
a) la mise en contact d'un fluide de culture filtré, contenant la glycoprotéine biologiquement active devant être isolée, avec une matrice chromatographique portant des radicaux dihydroxyboronyle, équilibrée au préalable avec un premier tampon d'équilibrage,
b) l'élution avec un premier tampon d'élution et la mise en contact de cet éluat directement avec une matrice échangeuse d'anions portant des groupes fonctionnels ammonium quaternaire,
c) l'élution de la glycoprotéine avec un deuxième tampon d'élution et sa collecte.

3. Procédé pour la purification d'érythropoïétine, qui comprend :
a) la mise en contact d'un fluide de culture filtré, contenant la glycoprotéine devant être isolée, avec un support chromatographique portant des radicaux dihydroxyboronyle, équilibré au préalable avec un premier tampon d'équilibrage,
b) l'élution avec un premier tampon d'élution,
c) la mise en contact de cet éluat directement avec une matrice échangeuse d'anions portant des groupes fonctionnels ammonium quaternaire,
d) l'élution avec un deuxième tampon d'élution,
e) l'ultrafiltration sur une membrane de séparation de 5 000 à 25 000 Da,
f) éventuellement la diafiltration pour échanger le tampon avec un tampon convenant pour l'étape suivante, et
g) la filtration sur gel sur une résine de séparation ayant une plage de séparation entre 5 000 et 250 000 Da.

4. Procédé pour la purification d'érythropoïétine, qui comprend :
a) la mise en contact d'un fluide de culture filtré ajusté à un pH de 7,5 à 9,0 avec un support chromatographique portant des radicaux dihydroxyboronyle équilibré avec un premier tampon d'équilibrage qui est un tampon aqueux à une concentration de 25 à 100 mM, avec une force ionique entre 2 et 20 mS/cm² et un pH entre 7,5 et 9,0,
b) le lavage subséquent avec le premier tampon d'équilibrage et ensuite avec le premier tampon d'équilibrage contenant de 10 à 100 mM d'une substance de faible masse moléculaire contenant un 1,2-cis-diol,
c) l'élution avec un premier tampon d'élution qui est un tampon aqueux ayant un pH entre 7,5 et 11,0 contenant un composé ayant des groupes 1-hydroxy-2-amino à une concentration de 20 à 200 mM, éventuellement en présence d'un agent chaotrope à raison de 2 à 8 M, d'un accepteur de cyanate à raison de 2 à 40 mM, et d'un tensioactif à raison de 0,01 % à 0,1 % (p/p),
d) la mise en contact de cet éluat avec une matrice échangeuse d'anions portant des groupes fonctionnels ammonium quaternaire équilibrée dans un deuxième tampon d'équilibrage qui est un tampon aqueux ayant un pH entre 7,5 et 11,0 et de 0,01 % à 0,1 % (p/p) d'un tensioactif,
e) le lavage subséquent avec le deuxième tampon d'équilibrage et le même tampon contenant de plus jusqu'à 50 mM de sel,
f) l'élution avec un deuxième tampon d'élution ayant un pH entre 7,5 et 11,0 en présence de 0,01 % à 0,1 % (p/p) d'un tensioactif et de 150 à 350 mM de sel,
g) l'ultrafiltration sur une membrane de séparation de 5 000 à 30 000 Da,
h) éventuellement la diafiltration pour échanger le tampon en un tampon qui est convenable pour l'étape suivante de filtration sur gel, et
i) la filtration sur gel sur une résine de séparation ayant une plage de séparation entre 5 000 et 250 000 Da.

5. Procédé selon l'une quelconque des revendications 1, 2, 3 et 4, dans lequel le support chromatographique portant des radicaux dihydroxyboronyle est un agarose au boronate de phényle.

6. Procédé selon l'une quelconque des revendications 1, 2, 3 et 4, dans lequel le premier tampon d'équilibrage est choisi parmi la glycine, le phosphate, un bicarbonate de trialkylammonium et l'acide 4-(2-hydroxyéthyl)-1-pipérazinoéthanesulfonique.

7. Procédé selon la revendication 6, dans lequel le premier tampon d'équilibrage est l'acide 4-(2-hydroxyéthyl)-1-pipérazinoéthanesulfonique à une concentration d'environ 0,05 M.

8. Procédé selon l'une quelconque des revendications 1 à 7, dans lequel le pH du premier tampon d'équilibrage est d'environ 8,5.

9. Procédé selon l'une quelconque des revendications 1 et 4 à 8, dans lequel la substance de faible masse moléculaire contenant un 1,2-cis-diol est choisie parmi les polyols à petite chaîne ouverte tels que le sorbitol, le mannitol, l'adonitol, l'arabitol, le glycérol, l'érythritol, et le cis-inositol, et les monosaccharides à chaîne fermée tels que le ribose et le mannose.

10. Procédé selon la revendication 9, dans lequel la substance de faible masse moléculaire contenant un 1,2-cis-diol est le sorbitol.

11. Procédé selon la revendication 9 ou 10, dans lequel la substance de faible masse moléculaire contenant un 1,2-cis-diol est présente à une concentration d'environ 0,05 M.

12. Procédé selon l'une quelconque des revendications 1 et 4 à 11, dans lequel le deuxième tampon d'équilibrage contient un composé ayant des groupes 1-hydroxy-2-amino à une concentration de 0,02 à 0,2 M.

13. Procédé selon l'une quelconque des revendications 1 et 4 à 11, dans lequel le deuxième tampon d'élution contient un composé ayant des groupes 1-hydroxy-2-amino à une concentration de 0,05 M.

14. Procédé selon l'une quelconque des revendications 4 à 13, dans lequel le composé ayant des groupes 1-hydroxy, 2-amino est choisi parmi le 2-amino-2-hydroxyméthyl-1,3-propanediol, le bis(hydroxyméthyl)aminoéthane, la N-[2-hydroxy-1,1-bis(hydroxyméthyl)éthyl]glycine, et la N,N-bis(2-hydroxyéthyl)glycine.

15. Procédé selon l'une quelconque des revendications 4 à 14, dans lequel l'agent chaotrope est choisi parmi l'urée et ses dérivés, et la guanidine.

16. Procédé selon la revendication 15, dans lequel l'agent chaotrope est de l'urée 6 M.

17. Procédé selon l'une quelconque des revendications 4 à 16, dans lequel l'accepteur de cyanate est la glycine.

18. Procédé selon l'une quelconque des revendications 3 à 17, dans lequel la matrice échangeuse d'anions portant des groupes fonctionnels ammonium quaternaire est choisis parmi les matrices de cellulose microcristalline ou d'agarose réticulé portant des groupes fonctionnels diéthylaminoéthyle, triéthylaminométhyle ou triméthylaminométhyle.

19. Procédé selon la revendication 18, dans lequel la matrice échangeuse d'anions est le triméthylaminométhyl-agarose.

20. Procédé selon l'une quelconque des revendications 3 à 19, dans lequel la filtration sur gel est réalisée avec une matrice de gel de dextrane/acrylamide réticulé à pores contrôlés.
